# EUROPEAN PATENT APPLICATION

(11) **EP 4 530 983 A1**
(43) Date of publication of application: **02.04.2025**
(21) Application number: 24203061.7
(22) Date of filing: 26.09.2024
(51) Int. Cl.: G06T 7/62

(54) **INJECTOR FLUID DELIVERY TESTING SYSTEMS**

(30) Priority: 28.09.2023 US 202363586151 P; 25.09.2024 US 202418896616
(71) Applicant: Illinois Tool Works Inc., Glenview IL 60025 (US)
(72) Inventor: EUSTON, Lindsay, Glenview, 60025 (US); BORER, Kevin, Glenview, 60025 (US); GOLDFARB, Landon, Glenview, 60025 (US); PINTO, Theresa, Glenview, 60025 (US); SALERNO, Nick, Glenview, 60025 (US)
(74) Representative: HGF

(57) **Abstract**

Disclosed example injector testing systems include: a camera configured to observe a needle of an injector; and control circuitry configured to: determine, based on images captured by the camera, a duration of fluid delivery by the injector; and determine, based on the images captured by the camera, a needle extension length at a start of fluid delivery by the injector.

## Description

### RELATED APPLICATIONS

The present application claims the benefit of U.S. Provisional Patent Application Serial No. 63/586,151, filed September 28, 2023, entitled "INJECTOR FLUID DELIVERY TESTING SYSTEMS." The entirety of U.S. Patent Application Serial No. 63/586,151 is expressly incorporated herein by reference.

### FIELD OF THE DISCLOSURE

This disclosure relates generally to injection device testing, and more particularly, to injector fluid delivery testing systems.

### BACKGROUND

Injection testing systems may test one or more aspects of injection devices, including autoinjectors, for aspects such as cap removal force, plunger actuation force, injection depth, needle retraction, and/or delivered dose.

### SUMMARY

Injector fluid delivery testing systems are disclosed, substantially as illustrated by and described in connection with at least one of the figures, as set forth more completely in the claims.

### BRIEF DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIG. 1 is an example injection testing system to perform fluid delivery testing of injection devices, in accordance with aspects of this disclosure.
FIG. 2 is a block diagram of an example injection testing system, in accordance with aspects of this disclosure.
FIG. 3 is a front elevation view of an example implementation of elements of the injection testing system of FIG. 2.
FIG. 4 illustrates an example image that may be captured by the camera of FIGS. 2 and 3 prior to actuation of an injector.
FIG. 5 illustrates an example image that may be captured by the camera of FIGS. 2 and 3 following extension of the needle of the injector and prior to a start of fluid delivery.
FIG. 6 illustrates an example image that may be captured by the camera of FIGS. 2 and 3 during fluid delivery by the injector.
FIG. 7 illustrates an example image that may be captured by the camera of FIGS. 2 and 3 when a last drop is adhered to the needle at an end of fluid delivery by the injector.
FIG. 8 illustrates an example image that may be captured by the camera of FIGS. 2 and 3 after the last drop of fluid is blown from the needle.
FIGS. 9A and 9B illustrate a flowchart representative of an example method to perform measurements during fluid delivery by an injector.

The figures are not necessarily to scale. Wherever appropriate, similar or identical reference numerals are used to refer to similar or identical components.

### DETAILED DESCRIPTION

Injection needle testing devices that measure the dosage delivered by the injection needle, particularly by autoinjectors, include a flask or other container positioned to capture and measure the fluid expelled from the needle. Some tests involve determining or verifying that a specified amount of fluid is delivered and/or that fluid is delivered to a specific depth (e.g., relative to a predetermined point on the injector).

Conventional methods of measuring a quantity of fluid delivery involve determining the start and end times of fluid delivery by measuring changes in mass using a scale. For instance, a collection container captures expelled fluid, and a scale is repeatedly monitored to determine times during which the mass changes (indicating expelling of fluid from the injector).

Conventional methods of determining whether fluid is delivered to a specific depth include placing a membrane at or near a threshold depth, such that fluid which is delivered by the injector outside of the target depth is prevented, by the membrane, from being collected for measurement.

The conventional techniques discussed above are expensive and time-consuming to implement, at least in part because the membrane may require regular replacement and/or positioning. Additionally, monitoring of fluid delivery via mass measurements may be relatively inaccurate.

Disclosed example injection fluid delivery testing systems and methods provide more reliable and accurate injector fluid delivery testing by analyzing images of the injector needle. Disclosed example systems and methods measure the needle extension length and detect the starting and stopping of fluid expulsion from the injector needle. By measuring the needle length using the camera, disclosed example systems and methods may determine the needle extension length both at the time the fluid delivery begins and can monitor the needle extension length over the entire duration of fluid delivery, without requiring the membrane involved in conventional methods.

Additionally, disclosed example systems and methods enable tracability of results by storing and/or associating with the test results the images captured at designated points during the testing and/or used to determine designated events during the testing. Examples of such designated points or events may include initiation of actuation, prior to the start of fluid delivery, the detected start of fluid delivery, the detected end of fluid delivery, intermediate starts and/or stops in fluid delivery, and/or the detection of a last drop of fluid.

The terms "expelling" and "delivering" are used interchangeably herein with respect to fluid being output from a needle of an injector.

Disclosed example injector testing systems include: a camera configured to observe a needle of an injector; and control circuitry configured to: determine, based on images captured by the camera, a duration of fluid delivery by the injector; and determine, based on the images captured by the camera, a needle extension length at a start of fluid delivery by the injector.

Some example injector testing systems further include a positioning surface configured to contact a body of the injector and having an aperture through which the needle extends. In some example injector testing systems, the control circuitry is configured to determine the needle extension length based on a location of the positioning surface, a location of the body, or a predetermined point. Some example injector testing systems further include an injector positioner configured to position the injector in contact with the positioning surface. Some example injector testing systems further include an injector actuator configured to actuate the injector to expel the fluid of the injector via the needle.

In some example injector testing systems, the control circuitry is configured to determine the duration of fluid delivery based on at least one of counting a number of frames in which fluid is observed in the images or monitoring a time during which fluid is observed in the images. In some example injector testing systems, the control circuitry is configured to analyze the images captured by the camera to detect at least one of a start of the duration of fluid delivery or an end of the duration of fluid delivery based on detecting a presence of a stream of fluid expelled from the needle. Some example injector testing systems further include a blowoff nozzle configured to direct gas toward the needle, in which the control circuitry is configured to control the blowoff nozzle to direct the gas toward the needle based on detecting an end of the stream of fluid.

Some example injector testing systems further include a scale configured to measure a mass of the expelled fluid. In some example injector testing systems, the control circuitry is configured to control the scale to determine the mass of the expelled fluid following the controlling of the blowoff nozzle to direct the gas toward the needle. In some example injector testing systems, the control circuitry is configured to determine whether the injector satisfies the predetermined injection criteria based on whether duration of fluid delivery satisfies a threshold.

In some example injector testing systems, the control circuitry is configured to determine whether the injector satisfies the predetermined injection criteria based on whether the needle extension length at the start time is at least a threshold length. Some example injector testing systems further include a second camera configured to record video of the needle of the injector.

In some example injector testing systems, the control circuitry is configured to determine the needle measurement location based on a predetermined search area within the images captured by the camera. In some example injector testing systems, the control circuitry is configured to determine the duration of fluid delivery as a total observed fluid delivery time or as a total start to end time. In some example injector testing systems, the control circuitry is configured to monitor, based on the images captured by the camera, the needle extension length from the start time to the end time.

In some example injector testing systems, control circuitry is configured to determine the duration of fluid delivery by: determining, based on images captured by the camera, at least one of a start time or a starting image frame at which fluid begins to be expelled from the needle; and determining, based on images captured by the camera, at least one of an end time or an ending image frame at which the needle has completed expelling of the fluid. In some example injector testing systems, the control circuitry is configured to determine the needle extension length by determining, based on images captured by the camera, a needle measurement location with respect to a reference location at the start of fluid delivery.

In some example injector testing systems, the control circuitry is configured to determine, based on the duration and the needle extension length at the start of fluid delivery, whether the injector satisfies predetermined injection criteria. In some example injector testing systems, the control circuitry is configured to determine whether the injector satisfies the predetermined injection criteria further based on whether the needle extension length between the start of the fluid delivery and an end of the fluid delivery satisfies a threshold length.

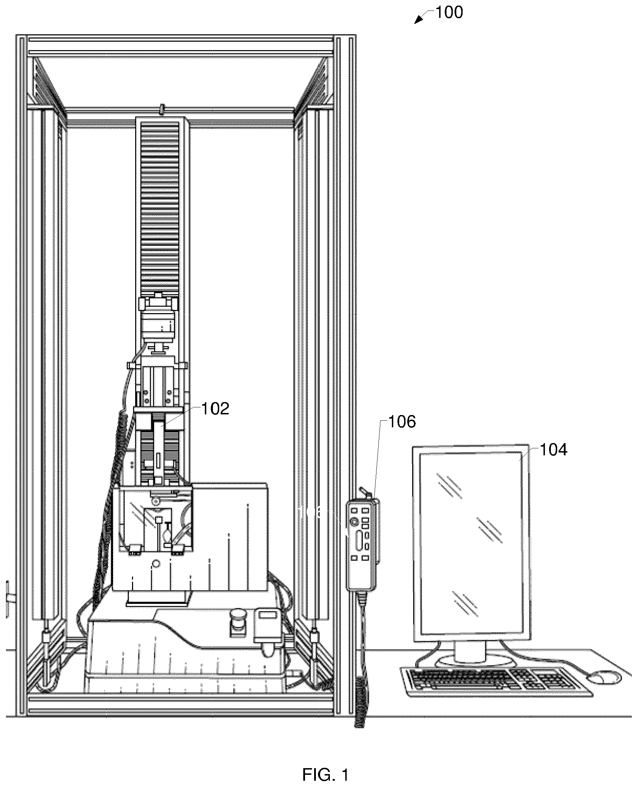

FIG. 1 is an example injection testing system 100 to perform testing on injection devices, such as an autoinjector 102. The example injection testing system 100 may be configured, for example, to perform some or all tests to evaluate requirements of the ISO 11608-5 standard. The example injection testing system 100 may be, for example, a universal testing system configured for injection testing.

The example injection testing system 100 of FIG. 1 includes positioning device(s) (e.g., to position the autoinjector 102 in one or more positions and/or orientations for automated testing), actuator(s) (e.g., to actuate components of the autoinjector 102, to actuate the positioning device(s), to position and/or orient test devices, etc.), and/or sensors to measure aspects of the autoinjector 102 during testing (e.g., load sensors to measure actuation force(s), auditory sensors to detect audible events), mass scales to measure an expelled dose, displacement and/or position sensors to trigger testing steps and/or measure displacement of components of the autoinjector 102, etc.). The example injection testing system 100 further includes one or more user interface devices, such as display 104 and input devices 106.

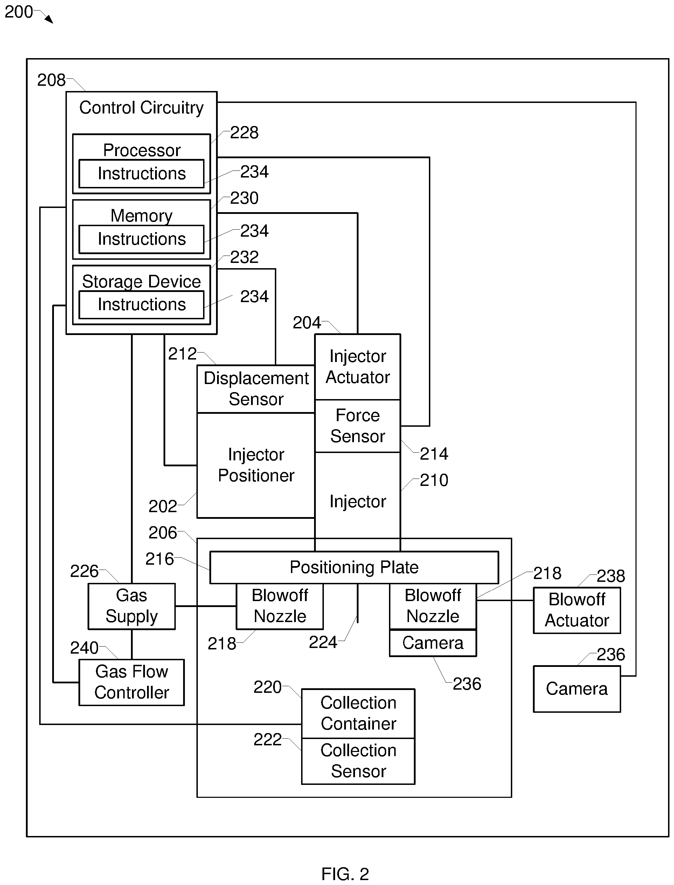

FIG. 2 is a block diagram of an example injection testing system 200 including an injection needle blowoff apparatus. The example injection testing system 200 may be used to implement some or all of the components of the injection testing system 100 of FIG. 1.

The example injection testing system 200 includes an injector positioner 202, an injector actuator 204, an injection collector 206, and control circuitry 208. The injector positioner 202 positions and/or orients an injector 210 (e.g., an autoinjector) for one or more tests in the injection testing system 200. For example, the injector positioner 202 may grasp the injector 210 and move and/or rotate the injector 210 for testing. The injector positioner 202 and/or the position of the injector 210 may be measured by one or more displacement sensor(s) 212, which provides displacement and/or position information to the control circuitry 208.

The injector actuator 204 actuates one or more aspects of the injector 210, such as a plunger or other injection mechanism of the injector 210. The force applied by the injector actuator 204 may by measured by a force sensor 214, which provides force measurements to the control circuitry 208.

The injection collector 206 includes a positioning surface (e.g., a positioning plate 216), blowoff nozzles 218, and a collection container 220. The collection container 220 and the injector 210 are positioned such that, when the injector 210 is actuated to expel fluid contained in the injector 210, the fluid is expelled into the collection container 220. A collection sensor 222 measures the mass and/or volume collected in the collection container 220, and provides a mass or volume measurement to the control circuitry 208. An example collection sensor 222 may be a scale, which may be calibrated for the presence of the collection container 220.

The positioning plate 216 allows a needle 224 of the injector 210 to extend through the positioning plate 216 toward the collection container 220. The positioning plate 216 may block the body of the injector 210 from extending through the positioning plate 216 using appropriately sized apertures for the needle 224 and body of the injector 210. To test the dispensing of the contained fluid, the injector positioner 202 may position the injector 210 to contact or abut the positioning plate 216, such that the needle 224 extends through the aperture of the positioning plate 216. When the injector 210 is positioned, the injector 210 may be actuated (e.g., manually, or automatically via the injector actuator 204) to expel the contents of the injector 210 into the collection container 220.

While the examples disclosed herein use the positioning plate 216 as a loading surface, other examples may use different types of loading surfaces against which the injector 210 can be actuated to expose the needle 224 and/or expel the contents of the injector 210. For example, rods or other structural members which are positioned to contact a body of the injector 210 on a top side of the loading surface, and to avoid obstructing the needle 224 may be used. In some such examples, the blowoff nozzles 218 may be coupled to another surface, or otherwise adjustably supported, within the injection collector 206 adjacent the bottom side of the loading surface and/or adjacent the location the needle 224.

At the completion of actuation of the injector 210, the blowoff nozzles 218 are controlled to blow the last drop of fluid from at or near the tip of the needle into the collection container 220. A gas supply 226 provides a gas, such as nitrogen or air, to the blowoff nozzles 218. The gas supply 226 may be, for example, a compressed gas source, a pneumatic pump, or a blower. The blowoff nozzles 218 may be positioned and/or oriented to adjust a location at which the blowoff gas strikes the needle 224, and/or may be positioned and/or oriented to cause the blowoff gas to strike the needle 224 over a range of needle lengths. In some examples, the blowoff nozzles 218 are omitted.

The example control circuitry 208 may be a general-purpose computer, a laptop computer, a tablet computer, and/or any other type of processing system configured to communicate with the sensors and actuators of the injection testing system 200. For example, the control circuitry 208 includes a processor 228, memory 230, and a storage device 232. The example processor 228 may be any general purpose central processing unit (CPU) from any manufacturer. In some other examples, the processor 228 may include one or more specialized processing units, such as RISC processors with an ARM core, graphic processing units, digital signal processors, and/or system-on-chips (SoC). The processor 228 executes machine readable instructions 234 that may be stored locally at the processor (e.g., in an included cache or SoC), in the memory (e.g., a random access memory or other volatile memory, a read only memory or other non-volatile memory such as FLASH memory, and/or in the storage device 232. The example storage device 232 may be a hard drive, a solid state storage drive, a hybrid drive, a RAID array, and/or any other mass data storage device.

The control circuitry 208 communicates with one or more cameras 236. The example cameras 236 are digital cameras, which may have at least a threshold framerate to facilitate determination of fluid delivery times with at least a threshold precision. The one or more cameras 236 are arranged to observe the needle 224, including the range of needle extension lengths that may be used by the injector 210. In some examples, a first camera takes still images at a predetermined rate and/or in response to events, and a second camera captures video at a predetermined frame rate.

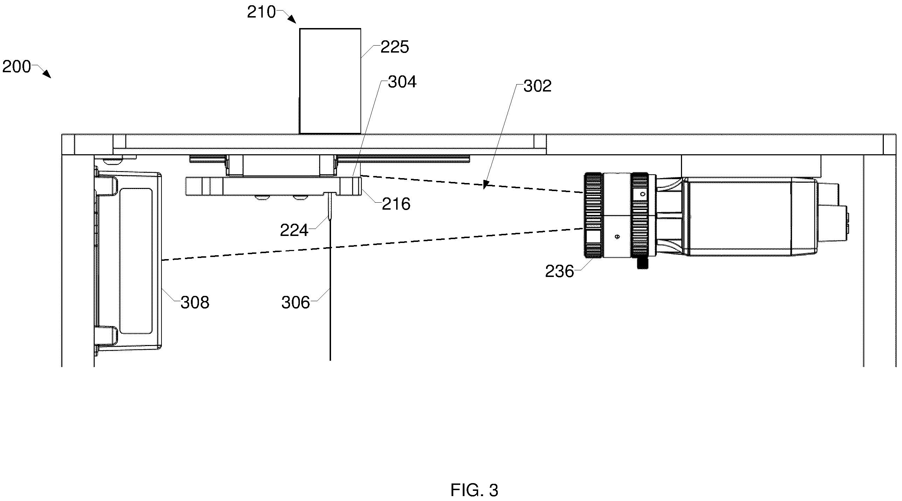

FIG. 3 is a front elevation view of an example implementation of elements of the injection testing system 200 of FIG. 2. The needle 224 of the injector 210 and the camera 236 are shown in FIG. 3, in which the camera 236 is arranged to observe the extension of the needle 224 (e.g., the needle extension occurs within a field of view 302 of the camera 236). A body 225 of the injector 210 is shown in contact with a positioning plate 216. An upper surface 304 of the positioning plate 216 provides a positioning surface. The body 225 of the injector 210 makes contact with the upper surface 304 to provide a consistent measurement location for different types of injectors.

Based on the images captured by the camera 236, the control circuitry 208 determines a duration of fluid delivery by the injector 210 and determines the needle extension length at a start of fluid delivery by the injector 210. For example, the control circuitry 208 may implement machine vision techniques to identify and measure the needle 224 and/or fluid 306 expelled from the needle 224 from images (e.g., video frames) captured by the camera 236.

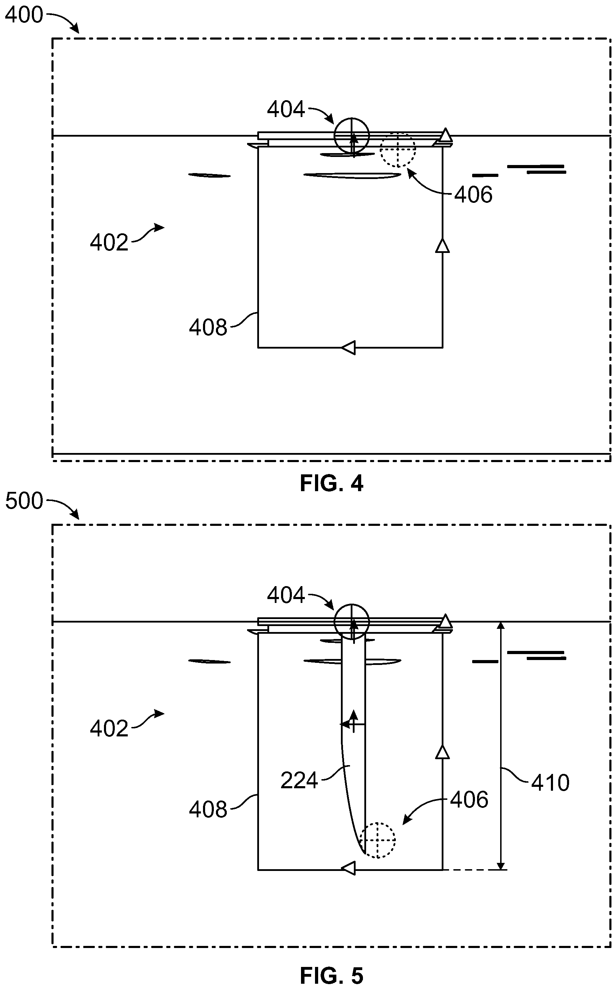

To detect and/or measure the needle 224, the control circuitry 208 may first determine a background image. For example, a screen 308 background may be positioned within the field of view 302. The screen 308 may be a light or dark color, or a light source, to provide the desired contrast with the needle 224 and/or the fluid 306. FIG. 4 illustrates an example image 400 that may be captured by the camera 236 of FIGS. 2 and 3 prior to actuation of an injector 210. The image 400 may be a filtered version of the image generated by the camera 236, such as to reduce colorization, enhance or reduce contrast, sharpen or blur edges, and/or any other desired filtering.

The example image 400 includes the background 402, a detected reference location 404, and a detected measurement location 406. The background 402 is based on the screen 308. The detected reference location 404 may be identified by the control circuitry 208 using a user-defined area and/or by observing and recognizing the positioning plate 216, the body 225 of the injector 210, and/or any other reference point or positioning surface in the image 400.

The detected measurement location 406 is determined by the control circuitry 208 based on the image 400. In the example image 400, the needle 224 is not observed within the image (e.g., because the needle 224 is not yet extended from the injector 210). As discussed below, the control circuitry 208 updates the measurement location 406 in response to detecting the needle 224, such as at a predetermined feature of the needle (e.g., the tip of the needle 224, the opening of the needle 224, etc.).

The control circuitry 208 may search for the needle measurement location within a predetermined needle search area 408 within the images generated by the camera 236. The needle search area 408 may be a user-selected, calibrated, determined via machine learning, and/or otherwise determined area within the images captured by the camera 236. The control circuitry 208 may use the needle search area 408 for exclusive searching for the needle 224 or as primary searching for the needle 224. In some examples, in the absence of detection of the needle 224, the control circuitry 208 sets or calculates the measurement location 406 to be a predetermined point in the image 400 and/or a predetermined point of the needle search area 408 (e.g., a top of the needle search area 408). The measurement location may be a predetermined location, a marked location on the needle 224, and/or calculated point on the needle 224 at which the length is measured with respect to the reference location 404.

To determine the duration of fluid delivery by the injector 210, the control circuitry 208 determines a start of the fluid delivery and an end of the fluid delivery. For example, fluid delivery may be identified in the images, and/or distinguished from the needle 224, based on an amount of contrast between the fluid and the background. Whereas the needle 224 may have a higher degree of contrast with the background, the fluid 306 may have less, but still identifiable, contrast with the background.

FIG. 5 illustrates an example image 500 that may be captured by the camera 236 of FIGS. 2 and 3 following extension of the needle 224 of the injector 210, and prior to a start of fluid delivery. In the example image 500, the control circuitry 208 detects the needle 224 based on a contrast between the needle 224 and the background 402. The control circuitry 208 determines the measurement location 406 based on, for example, a lowest detected point of the needle 224 that is at least a threshold width (e.g., in pixels or units of length). In some examples, the control circuitry 208 may use additional parameters to identify an object in the image 500 as the needle 224, such as having at least a threshold width at a specific point and/or any point along a visible length of the object. In some examples, the measurement location 406 is bounded by the needle search area 408. The measurement location 406 and/or the needle search area 408 may be limited or defined based on a user input specifying a nominal needle length (e.g., from manufacturer specifications, based on prior testing, etc.) and/or based on lower and/or upper limits on the needle length (e.g., from manufacturer specifications based on prior testing, etc.).

Using the measurement location 406 and the reference location 404, the control circuitry 208 determines a needle extension length 410. The control circuitry 208 may repeatedly and/or continuously update the needle extension length 410 to determine the needle extension length 410 at a start of fluid delivery by the needle 224 and/or to monitor the needle extension length 410 for the duration of fluid delivery by the needle 224 and/or for the duration of the injection event.

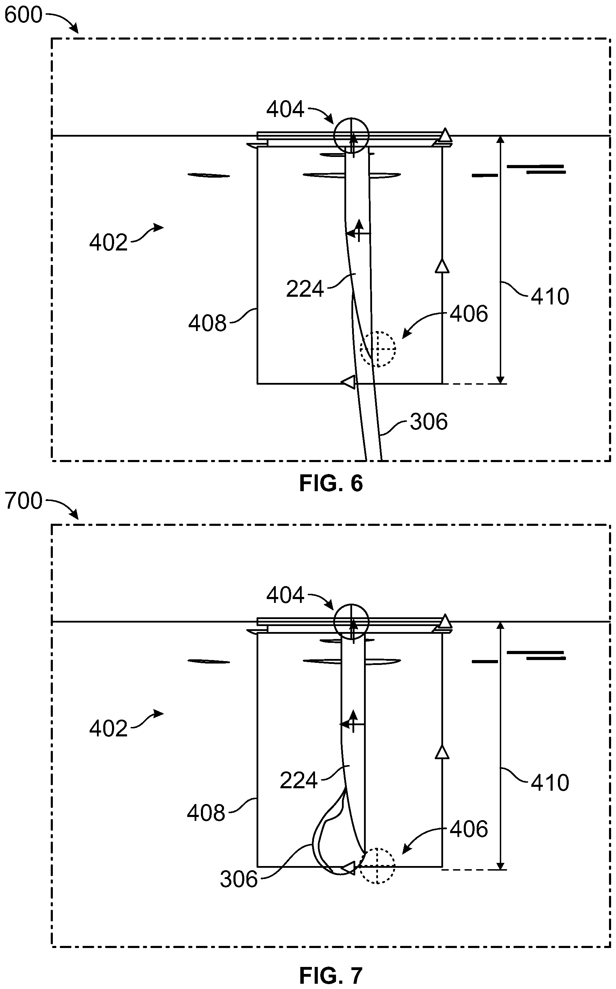

FIG. 6 illustrates an example image 600 that may be captured by the camera 236 of FIGS. 2 and 3 during fluid delivery by the injector 210. The control circuitry 208 identifies the presence of the fluid 306, such as a droplet or stream, in the image 600. For example, the fluid 306 may be detected based on edge detection (e.g., higher contrast at the edges of the fluid 306) and/or contrast between the fluid 306 and the background 402.

At a first frame or image in which the fluid 306 is detected, the example control circuitry 208 identifies the start of fluid delivery. The control circuitry 208 may start a timer or store an identifier of a particular frame (e.g., a frame number). The control circuitry 208 may continue to run the timer and/or count frames while the fluid 306 is observed to determine a duration of the fluid delivery.

FIG. 7 illustrates an example image 700 that may be captured by the camera 236 of FIGS. 2 and 3 when a last drop of the fluid 306 is adhered to the needle 224 at an end of fluid delivery by the injector 210. The control circuitry 208 may detect the last drop, or distinguish the last drop from a stream of the fluid 306, based on the width and/or length of the detected fluid in the image 700.

In response to detecting the last drop, the control circuitry 208 may trigger the gas supply 226 and/or the blowoff actuator 238 to perform a blowoff of the last drop from the needle 224, and/or may determine that the fluid delivery has ended. For example, the control circuitry 208 may store the frame number of the end of fluid delivery, and/or store a timer value at the end of fluid delivery. When using frame numbers, combined with the frame rate, the control circuitry 208 may calculate an approximate time between the fluid delivery starting frame and the fluid delivery ending frame to determine the duration of fluid delivery.

In some examples, the control circuitry 208 may identify intermediate breaks in the fluid delivery, and subtract the breaks from the total duration to determine a total observed fluid delivery time (e.g., exclusive of the breaks in the fluid delivery). In other examples, the control circuitry 208 determines the duration as a total start to end time of the fluid delivery, which is in inclusive of any breaks in the fluid delivery.

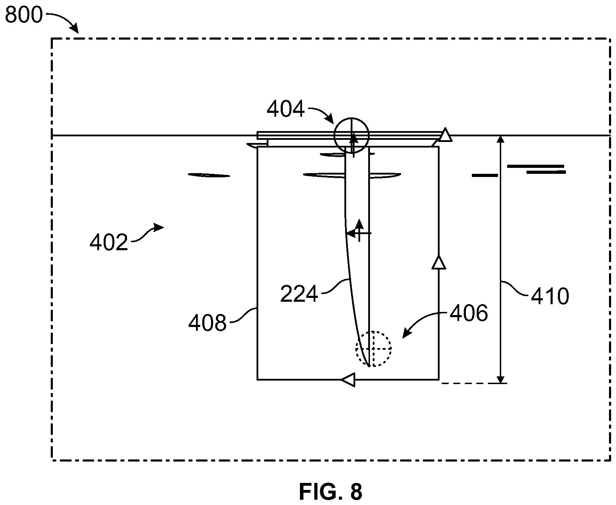

FIG. 8 illustrates an example image 800 that may be captured by the camera of FIGS. 2 and 3 after the last drop of fluid is blown from the needle 224. The control circuitry 208 may use the identification of removal of the last drop of fluid as a trigger to store the measured mass (or volume) of the fluid in the collection container 220 (via the collection sensor).

In some examples, the control circuitry 208 may omit the blowoff to reduce evaporation of the collected fluid in the collection container 220. In cases in which the collected fluid is more susceptible to evaporation, omitting the blowoff and triggering measurement of the collected fluid in response to detection of the last drop (FIG. 7) may result in a more accurate measurement of fluid delivered by the injector 210.

Upon completion of the actuation of the injector 210 and delivery of the fluid 306, the example control circuitry 208 may compare one or more of the duration of fluid delivery, the needle extension length 410 at the start of fluid delivery, the needle extension length 410 over the duration of the fluid delivery, the delivered mass and/or volume of fluid, and/or any other measured parameters, to corresponding thresholds to determine whether the injector 210 satisfies predetermined injection criteria. The injection criteria may be specific to a particular injector 210, and/or may be set by a standard such as the ISO 11608-5 standard. For example, the control circuitry 208 may determine whether the calculated duration of fluid delivery (whether inclusive or exclusive of breaks in fluid delivery) satisfies an upper limit and/or a lower limit on the duration. Additionally or alternatively, the control circuitry 208 may determine whether the needle extension length 410 satisfies a threshold length (e.g., a minimum length, a length range) at the start of fluid delivery and/or over the duration of fluid delivery. The control circuitry 208 may also determine whether the delivered fluid is at least a threshold mass and/or volume.

Based on the comparison(s), the control circuitry 208 may store and/or output an indication of the comparison(s). For example, the control circuitry 208 may output alerts for criteria that are not satisfied.

In some examples, the control circuitry 208 may determine a modified dose as a dose that is delivered to a designated therapeutic zone. For example, in the event that the injector 210 begins to expel fluid while the needle 224 is outside of the therapeutic zone, the control circuitry 208 ignores, or corrects for, that portion of the fluid that is expelled outside of the therapeutic zone, while measuring the fluid that is expelled within the therapeutic zone. In some such examples, the control circuitry 208 obtains or determines a first timestamp when the needle 224 extends into the therapeutic zone, and correlates the timestamp to a measurement value obtained from the collection sensor 222. The control circuitry 208 then obtains or determines a second timestamp when the flow ends, or the needle crosses out of the therapeutic zone, and correlates the second timestamp to a second measurement value from the collection sensor 222. The difference between the timestamps corresponds to the delivery time, and the difference between the obtained measurement values determines the delivered fluid.

Additionally or alternatively, when a test procedure begins, the control circuitry 208 controls or commands the collection sensor 222 to zero (or tare) the scale for each frame, until the needle 224 crosses into the therapeutic zone (regardless of whether there is fluid being expelled). The control circuitry 208 may then get the measurement value from the collection sensor 222 when either the flow ends or when the needle 224 leaves the therapeutic zone, which results in a measurement of expelled fluid that occurred within the therapeutic zone.

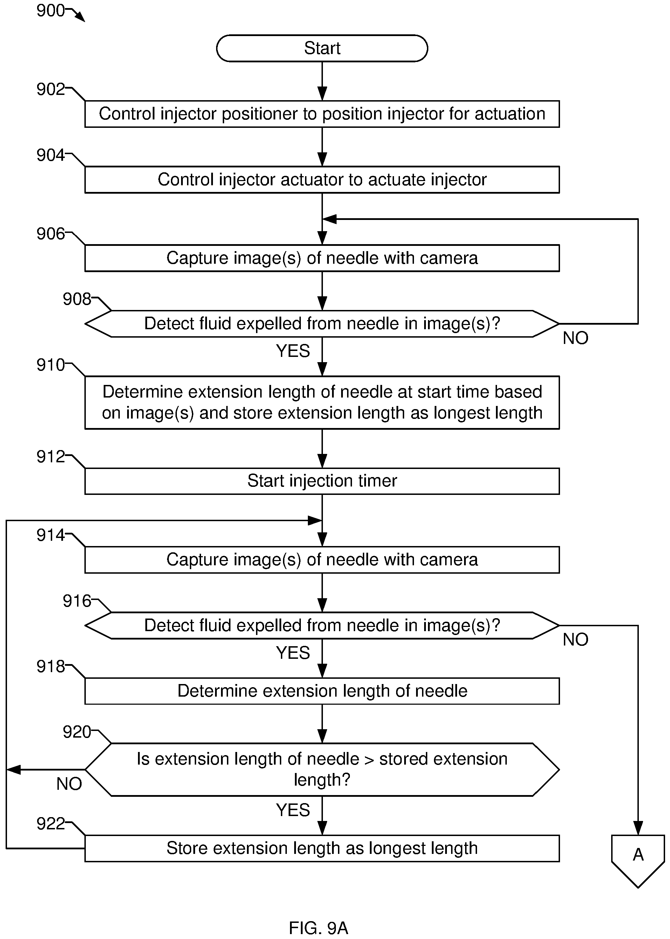

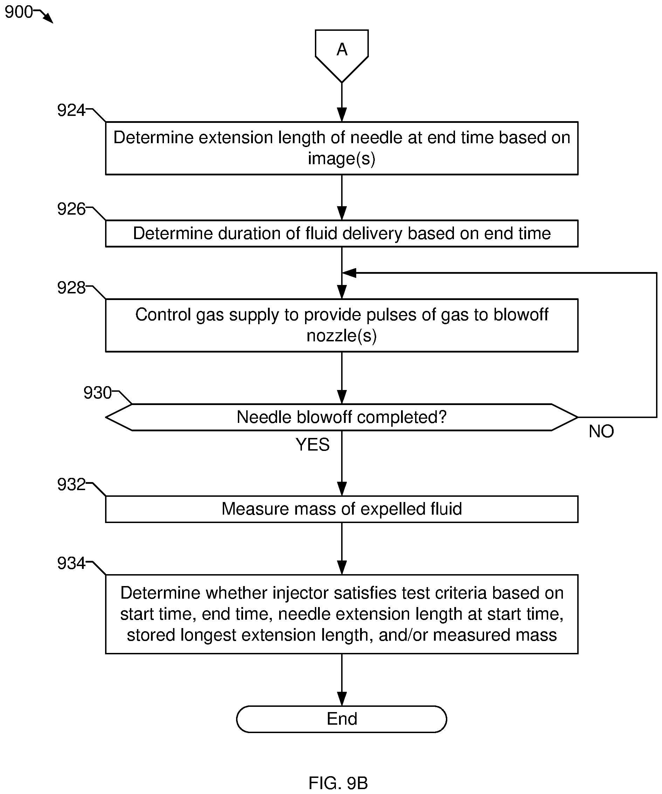

FIGS. 9A and 9B illustrate a flowchart representative of example machine readable instructions 900 which may be performed by the control circuitry 208 of FIG. 2 to perform measurements during fluid delivery by an injector 210. The example instructions 900 may implement the instructions 234 of FIG. 2 and/or be stored in the memory 230 and/or storage device 232, and executed by the processor 228.

At block 902, the control circuitry 208 controls the injector positioner 202 to position the injector 210 in contact with a positioning surface (e.g., the positioning plate 216). At block 904, the control circuitry 208 controls the injector actuator 204 to actuate the injector 210. For example, the injector actuator 204 may actuate a plunger, button, or other trigger device of the injector 210 and/or push down on the body 225 of the injector 210 to activate a push trigger.

At block 906, the camera 236 captures image(s) of the needle 224. For example, the camera 236 may capture image(s) of the field of view 302 of the camera 236 to observe extension of the needle 224.

At block 908, the control circuitry 208 determines whether the expelling of fluid from the needle 224 can be detected in the images. For example, the control circuitry 208 may use machine vision techniques to determine whether the fluid 306 is observed in the images. If expelling of fluid is not detected (block 908), control returns to block 906.

If expelling of fluid is detected (block 908), at block 910 the control circuitry 208 determines the needle extension length 410 at a start time of fluid delivery. For example, the control circuitry 208 may identify the reference location 404 and the detected measurement location 406 of the needle 224 based on the images (e.g., using machine vision techniques). In some examples, the control circuitry 208 may maintain an array, table, or register of determined extension lengths for images of the needle captured by the camera in block 906. The extension length at the start time may be determined as the most recent measurement value prior to detecting fluid expelled from the needle at block 908.

At block 912, the control circuitry 208 starts an injection timer. The timer may be a clock or other register to track the duration of the fluid delivery by the injector 210. In some other examples, the control circuitry 208 may track frames instead of, or in addition to, running a timer.

At block 914, the control circuitry 208 captures additional images via the camera 236. At block 916, the control circuitry 208 determines whether fluid 306 is still being expelled from the needle 224 in the captured images. Block 916 may be implemented in a similar or identical manner to block 908.

If fluid 306 is still being expelled from the needle 224 in the captured images (block 916), at block 918, the control circuitry 208 determines an extension length 410 of the needle 224. For example, the control circuitry 208 may identify the reference location 404 and the detected measurement location 406 of the needle 224 based on the images (e.g., using machine vision techniques).

At block 920, the control circuitry 208 determines whether the determined extension length of the needle 224 (block 918) is greater than a stored extension length. For example, the control circuitry 208 may store the longest observed extension length 410 (e.g., separately from the measured initial extension length). If the determined extension length of the needle 224 is greater than the stored extension length (block 920), at block 922 the control circuitry 208 stores the extension length as the longest observed length, and returns control to block 914 to continue monitoring.

Additionally or alternatively, the control circuitry 208 determines a shortest needle extension length 410 over the duration of the fluid delivery. For example, the control circuitry 208 may store the shortest observed needle extension length, and update the stored shortest observed needle extension length when a shorter needle extension length is observed.

Turning to FIG. 9B, if fluid 306 is no longer being expelled from the needle 224 in the captured images (e.g., the last drop is detected, or no fluid is detected) (block 916), at block 924 the control circuitry 208 determines the extension length 410 of the needle 224 at the end time, based on the images. Block 924 may be implemented in a similar manner as block 918.

In some examples, the control circuitry 208 may determine whether one or more injection end criteria have been met before determining that the injection is finished and proceeding to block 924. For example, injection end criteria may include determining whether at least a threshold amount of fluid has been expelled, at least a threshold time (or number of frames) has elapsed since the initial detection of fluid stoppage, and/or at least a threshold time (or number of frames) has elapsed since the start of the start of the injection (block 912), and/or any other criteria to determine that the injection is completed. A failure to meet the end criteria may cause the control circuitry 208 to revert control to block 914.

At block 926, the control circuitry 208 determines a duration of the fluid delivery based on the detected end of the fluid delivery in the images. In some other examples, the control circuitry 208 may monitor a number of frames of video by the camera 236 and determine the duration based on the number of frames and a known framerate of the camera 236.

At block 928, the control circuitry 208 controls the gas supply 226 to provide gas to blowoff nozzle(s) 218. For example the blowoff nozzles 218 may blow off a last drop of the fluid from the needle 224 for collection and measurement of mass. Blowing off may be implemented using one or more pulses of air (which may have any desired pulse length). In some other examples, blowoff may be implemented by the blowoff actuator 238 and/or omit the blowoff.

At block 930, the control circuitry 208 determines whether the needle blowoff is completed. For example, the control circuitry 208 may determine whether the last drop is still present in captured images. If needle blowoff is not completed (block 930), control returns to block 928. In some examples, the determination of the extension length at the end time (block 924) may occur following the needle blowoff at block 930.

When the needle blowoff is completed (block 930), at block 932 the control circuitry 208 measures a mass of the expelled fluid (e.g., via the collection sensor 222). At block 934, the control circuitry 208 determines whether the injector 210 satisfies one or more test criteria. For example, the test criteria may be compared to a start time and/or end time of fluid delivery, a duration of fluid delivery, a needle extension length at the start time, the stored longest needle extension length (and/or stored shortest needle extension length), and/or a measured mass. The control circuitry 208 may store and/or output (e.g., display) the results of the comparison(s).

The example instructions 900 then end.

The present methods and systems may be realized in hardware, software, and/or a combination of hardware and software. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise an application specific integrated circuit or chip. Some implementations may comprise a non-transitory machine-readable (e.g., computer readable) medium (e.g., FLASH drive, optical disk, magnetic storage disk, or the like) having stored thereon one or more lines of code executable by a machine, thereby causing the machine to perform processes as described herein. As used herein, the term "non-transitory machine-readable medium" is defined to include all types of machine readable storage media and to exclude propagating signals.

As utilized herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x),

(y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present method and/or system has been described with reference to certain implementations, it will be understood by those skilled in the art that various changes may be made and equivalents may be substituted without departing from the scope of the present method and/or system. For example, block and/or components of disclosed examples may be combined, divided, re-arranged, and/or otherwise modified. In addition, many modifications may be made to adapt a particular situation or material to the teachings of the present disclosure without departing from its scope. Therefore, the present method and/or system are not limited to the particular implementations disclosed. Instead, the present method and/or system will include all implementations falling within the scope of the appended claims, both literally and under the doctrine of equivalents.

Certain embodiments of the invention are described in the following clauses:
Clause 1. An injector testing system, comprising:
   a camera configured to observe a needle of an injector; and
   control circuitry configured to:
      determine, based on images captured by the camera, a duration of fluid delivery by the injector; and
      determine, based on the images captured by the camera, a needle extension length at a start of fluid delivery by the injector.
Clause 2. The injector testing system as defined in clause 1, further comprising a positioning surface configured to contact a body of the injector and having an aperture through which the needle extends.
Clause 3. The injector testing system as defined in clause 2, wherein the control circuitry is configured to determine the needle extension length based on a location of the positioning surface, a location of the body, or a predetermined point.
Clause 4. The injector testing system as defined in clause 2, further comprising an injector positioner configured to position the injector in contact with the positioning surface.
Clause 5. The injector testing system as defined in clause 2, further comprising an injector actuator configured to actuate the injector to expel the fluid of the injector via the needle.
Clause 6. The injector testing system as defined in clause 1, wherein the control circuitry is configured to determine the duration of fluid delivery based on at least one of counting a number of frames in which fluid is observed in the images or monitoring a time during which fluid is observed in the images.
Clause 7. The injector testing system as defined in clause 1, wherein the control circuitry is configured to analyze the images captured by the camera to detect at least one of a start of the duration of fluid delivery or an end of the duration of fluid delivery based on detecting a presence of a stream of fluid expelled from the needle.
Clause 8. The injector testing system as defined in clause 7, further comprising a blowoff nozzle configured to direct gas toward the needle, wherein the control circuitry is configured to control the blowoff nozzle to direct the gas toward the needle based on detecting an end of the stream of fluid.
Clause 9. The injector testing system as defined in clause 8, further comprising a scale configured to measure a mass of the expelled fluid.
Clause 10. The injector testing system as defined in clause 9, wherein the control circuitry is configured to control the scale to determine the mass of the expelled fluid following the controlling of the blowoff nozzle to direct the gas toward the needle.
Clause 11. The injector testing system as defined in clause 1, wherein the control circuitry is configured to determine whether the injector satisfies the predetermined injection criteria based on whether duration of fluid delivery satisfies a threshold.
Clause 12. The injector testing system as defined in clause 1, wherein the control circuitry is configured to determine whether the injector satisfies the predetermined injection criteria based on whether the needle extension length at the start time is at least a threshold length.
Clause 13. The injector testing system as defined in clause 1, further comprising a second camera configured to record video of the needle of the injector.
Clause 14. The injector testing system as defined in clause 1, wherein the control circuitry is configured to determine the needle measurement location based on a predetermined search area within the images captured by the camera.
Clause 15. The injector testing system as defined in clause 1, wherein the control circuitry is configured to determine the duration of fluid delivery as a total observed fluid delivery time or as a total start to end time.
Clause 16. The injector testing system as defined in clause 1, wherein the control circuitry is configured to monitor, based on the images captured by the camera, the needle extension length from the start time to the end time.
Clause 17. The injector testing system as defined in clause 1, wherein the control circuitry is configured to determine the duration of fluid delivery by:
   determining, based on images captured by the camera, at least one of a start time or a starting image frame at which fluid begins to be expelled from the needle; and
   determining, based on images captured by the camera, at least one of an end time or an ending image frame at which the needle has completed expelling of the fluid.
Clause 18. The injector testing system as defined in clause 1, wherein the control circuitry is configured to determine the needle extension length by determining, based on images captured by the camera, a needle measurement location with respect to a reference location at the start of fluid delivery.
Clause 19. The injector testing system as defined in clause 1, wherein the control circuitry is configured to determine, based on the duration and the needle extension length at the start of fluid delivery, whether the injector satisfies predetermined injection criteria.
Clause 20. The injector testing system as defined in clause 19, wherein the control circuitry is configured to determine whether the injector satisfies the predetermined injection criteria further based on whether the needle extension length between the start of the fluid delivery and an end of the fluid delivery satisfies a threshold length.

## Claims

1. An injector testing system, comprising:
a camera configured to observe a needle of an injector; and
control circuitry configured to:
determine, based on images captured by the camera, a duration of fluid delivery by the injector; and
determine, based on the images captured by the camera, a needle extension length at a start of fluid delivery by the injector.

2. The injector testing system as defined in claim 1, further comprising a positioning surface configured to contact a body of the injector and having an aperture through which the needle extends.

3. The injector testing system as defined in claim 2, wherein the control circuitry is configured to determine the needle extension length based on a location of the positioning surface, a location of the body, or a predetermined point.

4. The injector testing system as defined in claim 2, further comprising an injector positioner configured to position the injector in contact with the positioning surface.

5. The injector testing system as defined in claim 2, further comprising an injector actuator configured to actuate the injector to expel the fluid of the injector via the needle.

6. The injector testing system as defined in claim 1, wherein the control circuitry is configured to determine the duration of fluid delivery based on at least one of counting a number of frames in which fluid is observed in the images or monitoring a time during which fluid is observed in the images.

7. The injector testing system as defined in claim 1, wherein the control circuitry is configured to analyze the images captured by the camera to detect at least one of a start of the duration of fluid delivery or an end of the duration of fluid delivery based on detecting a presence of a stream of fluid expelled from the needle.

8. The injector testing system as defined in claim 7, further comprising a blowoff nozzle configured to direct gas toward the needle, wherein the control circuitry is configured to control the blowoff nozzle to direct the gas toward the needle based on detecting an end of the stream of fluid.

9. The injector testing system as defined in claim 8, further comprising a scale configured to measure a mass of the expelled fluid, and optionally
wherein the control circuitry is configured to control the scale to determine the mass of the expelled fluid following the controlling of the blowoff nozzle to direct the gas toward the needle.

10. The injector testing system as defined in claim 1, wherein the control circuitry is configured to determine whether the injector satisfies the predetermined injection criteria based on whether duration of fluid delivery satisfies a threshold, or
wherein the control circuitry is configured to determine whether the injector satisfies the predetermined injection criteria based on whether the needle extension length at the start time is at least a threshold length.

11. The injector testing system as defined in claim 1, further comprising a second camera configured to record video of the needle of the injector, or
wherein the control circuitry is configured to determine the needle measurement location based on a predetermined search area within the images captured by the camera.

12. The injector testing system as defined in claim 1, wherein the control circuitry is configured to determine the duration of fluid delivery as a total observed fluid delivery time or as a total start to end time, or
wherein the control circuitry is configured to monitor, based on the images captured by the camera, the needle extension length from the start time to the end time.

13. The injector testing system as defined in claim 1, wherein the control circuitry is configured to determine the duration of fluid delivery by:
determining, based on images captured by the camera, at least one of a start time or a starting image frame at which fluid begins to be expelled from the needle; and
determining, based on images captured by the camera, at least one of an end time or an ending image frame at which the needle has completed expelling of the fluid.

14. The injector testing system as defined in claim 1, wherein the control circuitry is configured to determine the needle extension length by determining, based on images captured by the camera, a needle measurement location with respect to a reference location at the start of fluid delivery.

15. The injector testing system as defined in claim 1, wherein the control circuitry is configured to determine, based on the duration and the needle extension length at the start of fluid delivery, whether the injector satisfies predetermined injection criteria, and optionally
wherein the control circuitry is configured to determine whether the injector satisfies the predetermined injection criteria further based on whether the needle extension length between the start of the fluid delivery and an end of the fluid delivery satisfies a threshold length.
